(19) Europäisches Patentamt
European Patent Office
Office européen des brevets

(11) Veröffentlichungsnummer: **0 069 905**
**B1**

# (12) EUROPÄISCHE PATENTSCHRIFT

(45) Veröffentlichungstag der Patentschrift: **29.05.85**

(51) Int. Cl.⁴: **C 07 C 123/02**

(21) Anmeldenummer: **82105706.4**

(22) Anmeldetag: **28.06.82**

(54) **Verfahren zur Herstellung von Hydrazidinen.**

(30) Priorität: **09.07.81 DE 3127042**

(43) Veröffentlichungstag der Anmeldung:
**19.01.83 Patentblatt 83/3**

(45) Bekanntmachung des Hinweises auf die Patenterteilung:
**29.05.85 Patentblatt 85/22**

(84) Benannte Vertragsstaaten:
**AT BE CH DE FR GB IT LI NL**

(56) Entgegenhaltungen:
**EP - A - 0 069 883**

**JUSTUS LIEBIGS "Annalen der Chemie", Nr. 1 1975, VERLAG CHEMIE GMBH, Weinheim NEUNHOEFFER et al. "Synthese N-unsubstituierter Hydrazidine", Seiten 1120-1123**

(73) Patentinhaber: **BAYER AG, Konzernverwaltung RP Patentabteilung, D-5090 Leverkusen 1 Bayerwerk (DE)**

(72) Erfinder: **Bonse, Gerhard, Dr., Wolfskaul 3, D-5000 Köln 80 (DE)**
Erfinder: **Schmidt, Thomas, Dr., Ginsterweg 9, D-5657 Haan (DE)**

**EP 0 069 905 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues vorteilhaftes, von Iminoalkylethern ausgehendes Verfahren zur Herstellung von weitgehend bekannten Hydrazidinen.

Es ist bereits bekannt geworden, daß man Acethydrazidin als Hydrochlorid erhält, wenn man zunächst in einer ersten Stufe in eine vorgelegte Mischung von wasserfreiem Hydrazin in absolutem Ethanol Acetiminoethyletherhydrochlorid einträgt. Nach Isolierung des dabei in einer Ausbeute von 50% der Theorie gebildeten Acetamidrazonhydrochlorids wird dieses in einer zweiten Stufe mit in absolutem Ethanol gelösten wasserfreiem Hydrazin zu Acethydrazidinhydrochlorid umgesetzt [vgl. Mh. Chem. 63, 285–300 (1933)].

Es ist weiterhin bekannt geworden, daß man bestimmte Hydrazidine erhält, wenn man zunächst Iminoether in Amidiniumsalze von Carbonsäuren überführt und diese mit Hydrazin in Amidrazonsalze umwandelt; nach deren Isolierung wird in einer zweiten Stufe, nach Umsetzung mit wasserfreiem Hydrazin in wasserfreiem Lösungsmittel, die Reaktionsmischung bei vermindertem Druck (ca. 480 mbar) einige Zeit auf 40°C erwärmt und nach Entfernen des Lösungsmittels werden die entsprechenden Hydrazidinsalze erhalten (vgl. Liebigs Ann. Chem. 749, S. 16–23 (1971) und Liebigs Ann. Chem. 1975, S. 1120–1123.

Acethydrazidinhydrochlorid kann so ausgehend von Acetamidinhydrochlorid in einer Gesamtausbeute von 83% der Theorie erhalten werden.

Diese mehrstufigen Verfahren haben jedoch den Nachteil, daß neben einer Zwischenisolierung des Amidrazons weiterhin mit wasserfreiem Hydrazin in wasserfreiem Lösungsmittel umgesetzt werden muß. Dies bedeutet einen erheblichen technischen Aufwand; das Arbeiten mit wasserfreiem Hydrazin stellt darüberhinaus bei der technischen Synthese ein Sicherheitsrisiko dar.

Es wurde nun überraschend gefunden, daß man die weitgehend bekannten Hydrazidine der Formel

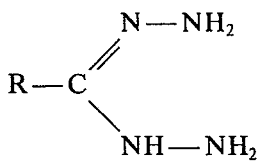

(I)

in welcher

R für gegebenenfalls substituiertes Alkyl, gegebenenfalls substituiertes Cycloalkyl, gegebenenfalls substituiertes Aryl oder gegebenenfalls substituiertes Aralkyl steht,

bzw. deren Säureadditionssalze, ausgehend von entsprechenden Iminoalkylethern in sehr guten Ausbeuten und in sehr reiner Form durch eine neue einstufige Verfahrensweise herstellen kann, indem man Iminoalkylether der Formel

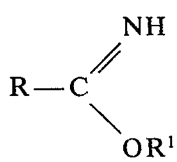

(II)

in welcher

R die oben angegebene Bedeutung hat, und
$R^1$ für gegebenenfalls substituiertes Alkyl oder gegebenenfalls substituiertes Cycloalkyl steht,

oder deren Säureadditionssalze mit Hydrazinhydrat ($NH_2-NH_2 \times H_2O$) in technischen (wasserhaltigen) Lösungsmitteln bei vermindertem Druck und bei Temperaturen zwischen −80 und +100°C umsetzt.

Das erfindungsgemäße Verfahren weist eine Reihe von Vorteilen auf. Neben deutlich höheren Ausbeuten ist es besonders zweckmäßig, die Gesamtreaktion als »Eintopfverfahren« durchzuführen. Weiterhin können anstelle von wasserfreiem Hydrazin und absoluten Lösungsmitteln Hydrazinhydrat und technische, d. h. wasserhaltige Lösungsmittel verwendet werden.

Die nach dem erfindungsgemäßen Verfahren herstellbaren Hydrazidine sind durch die Formel (I) allgemein definiert. In dieser Formel steht R vorzugsweise für gegebenenfalls substituiertes geradkettiges oder verzweigtes Alkyl mit 1–12 Kohlenstoffatomen, gegebenenfalls substituiertes Cycloalkyl mit 3–6 Kohlenstoffatomen, gegebenenfalls substituiertes Aryl mit 6 oder 10 Kohlenstoffatomen und für gegebenenfalls substituiertes Aralkyl mit 6 oder 10 Kohlenstoffatomen im Arylteil und 1–4 Kohlenstoffatomen im Alkylteil.

$R^1$ steht vorzugsweise für gegebenenfalls substituiertes geradkettiges oder verzweigtes Alkyl mit 1–12 Kohlenstoffatomen und gegebenenfalls substituiertes Cycloalkyl mit 3–6 Kohlenstoffatomen.

Die vorgenannten Reste können grundsätzlich durch solche Gruppen substituiert sein, die unter den

Reaktionsbedingungen nicht mit Hydrazinhydrat reagieren.

Beispielsweise können die Alkylreste durch Halogen (insbesondere Chlor oder Fluor) oder durch Alkoxy oder Alkylthio mit jeweils 1 bis 4 Kohlenstoffatomen substituiert sein.

Die Cycloalkylreste können beispielsweise durch Halogen (insbesondere Chlor oder Fluor), durch Alkyl, Alkoxy oder Alkylthio mit jeweils 1—4 Kohlenstoffatomen oder durch (gegebenenfalls selbst substituiertes) Phenyl substituiert sein.

Die Aryl- und Aralkylreste können beispielsweise durch Halogen (vorzugsweise Fluor, Chlor oder Brom), durch Nitro, durch Alkyl, Alkoxy und Alkylthio mit jeweils 1—4 Kohlenstoffatomen oder durch Halogenalkyl, Halogenalkoxy oder Halogenalkylthio mit jeweils 1—4 Kohlenstoffatomen und bis zu 5 gleichen oder verschiedenen Halogenatomen (insbesondere Fluor- und Chloratomen) substituiert sein.

Besonders bevorzugt sind diejenigen Verbindungen der Formel (I), in denen R für Methyl, Chlormethyl, Ethyl, n-Propyl, Isopropyl, n-Butyl, sek-Butyl, tert-Butyl, Cyclopropyl, Cyclobutyl, Cyclopentyl, Cyclohexyl, Phenyl oder Benzyl steht.

Verwendet man beispielsweise Acetiminoethyletherhydrochlorid und Hydrazinhydrat als Ausgangsstoffe, so kann der Reaktionsablauf durch das folgende zusammenfassende Formelschema wiedergegeben werden:

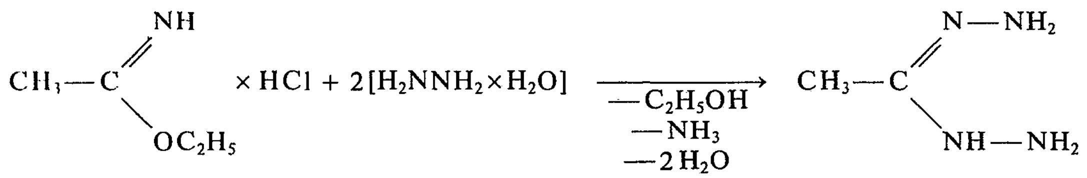


Die bei der Durchführung des erfindungsgemäßen Verfahrens als Ausgangsstoffe benötigten Iminoalkylether sind durch die Formel (II) allgemein definiert. In dieser Formel steht R vorzugsweise für diejenigen Reste, die bereits im Zusammenhang mit der Beschreibung der erfindungsgemäß herstellbaren Stoffe der Formel (I) vorzugsweise für diesen Substituenten genannt wurden.

Die Iminoalkylether der Formel (II) sowie ihre Säureadditionssalze sind allgemein bekannte Verbindungen der organischen Chemie, bzw. können sie nach bekannten Verfahren hergestellt werden (vergleiche Org. Synthesis Coll. Vol. I, S. 5 (1951); Beilstein Bd. 2, S. 182, 2, S. 245; 2/III, S. 451; 2/III, S. 618; 2/III, S. 675).

Als Beispiele seien genannt: Acetiminoethylether, Chloracetiminoethylether, Trichloracetiminomethylether, Propioniminoethylether, Butyriminoethylether, Valeriminoethylether sowie deren Hydrochloride.

Als Verdünnungsmittel kommen für die erfindungsgemäße Umsetzung inerte organische Lösungsmittel in Frage, die durchaus in Form technischer Lösungsmittel bis zu 20% Wasser enthalten können.

Hierzu gehören vorzugsweise Alkohole, wie Methanol, Ethanol, n-Propanol, Isopropanol, n-Butanol, Isobutanol, tert-Butanol, Glycol, Glycerin, Amylalkohol, Cyclohexanol, 2-Methyl-4-pentanol, Methylglycol, n-Hexanol, Isohexylalkohol, Isoheptylalkohol, n-Heptanol, Nonylalkohol, Dodecylalkohol oder Methylcyclohexanol; Ether wie Tetrahydrofuran, Dioxan oder Ethylenglycolmonomethylether; Amide, wie Dimethylformamid, Diethylformamid, Dimethylacetamid oder N-Methylpyrrolidon; Kohlenwasserstoffe, wie Benzol oder Toluol; sowie halogenierte Kohlenwasserstoffe, wie Methylenchlorid, Chloroform, Tetrachlorkohlenstoff, 1,2-Dichlorethan, 1,1,2-Trichlorethan, Trichlorethylen sowie Chlorbenzol. Es können auch entsprechende Lösungsmittelgemische verwendet werden.

Die Umsetzung wird bei vermindertem Druck durchgeführt, um das bei der Reaktion freigesetzte Ammoniak aus dem Reaktionsgemisch zu entfernen. Es ist zweckmäßig, im Druckbereich von etwa 0,1 bis etwa 800 mbar zu arbeiten; bevorzugt wird bei Drucken zwischen 50 und 300 mbar gearbeitet.

Die Reaktionstemperaturen können in einem größeren Bereich variiert werden und richten sich nach dem jeweils angewandten Druck. Die Umsetzung kann, wie oben angegeben, im Temperaturbereich von etwa —80 bis +100°C durchgeführt werden; bevorzugt arbeitet man zwischen —30 und +50°C. Das Verfahren kann kontinuierlich oder diskontinuierlich durchgeführt werden.

Bei der Durchführung des erfindungsgemäßen Verfahrens setzt man im allgemeinen auf 1 Mol eines Iminoalkylethers der Formel (II) oder eines entsprechenden Säure-Additionssalzes 2 bis 2,5 Mol, vorzugsweise 2 bis 2,3 Mol Hydrazinhydrat ein.

Die Isolierung der Verfahrensprodukte der Formel (I) erfolgt in üblicher Weise durch Entfernen des Lösungsmittels. Die nach dem erfindungsgemäßen Verfahren in hoher Reinheit und Ausbeute anfallenden Hydrazidine bzw. Hydrazidin-Säureadditionssalze können vorzugsweise ohne Isolierung als Lösung oder Suspension zu Folgeprodukten umgesetzt werden.

Hydrazidine sind wertvolle Synthesebausteine für zahlreiche Heterocyclen und hier insbesondere für herbizid wirksame as-Triazinone (vergleiche Liebigs Ann. Chem. 1976, S. 2206—2221; ibid. 1975, S. 1120—1123; Chem. Ber. 108, S. 3509—3517 (1975); Chem. Ber. 112, S. 1981—1990 (1979); DE-OS 2 224 161; DE-OS 2 556 835; DE-OS 3 102 318).

Die nachfolgenden Beispiele sollen die Erfindung näher erläutern.

## Herstellungsbeispiele

### Beispiel 1

Acethydrazidinhydrochlorid

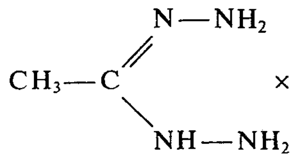

× HCl

In einen 1-Liter-Dreihalskolben werden 31,4 g (0,62 Mol) Hydrazinhydrat in 200 ml Ethanol eingetragen und ein verminderter Druck von 100 mbar eingestellt. Unter starkem Rühren wird nun eine Suspension von 38 g (0,31 Mol) Acetiminoethyletherhydrochlorid in 200 ml Ethanol bei 25°C zugesetzt und weitere zwei Stunden nachgerührt. Anschließend wird Ethanol im Vakuum abgezogen und man erhält nach dem Trocknen 36,9 g Acethydrazin-hydrochlorid als farblose Kristalle, Zersetzungspunkt 140–150°C, vom Gehalt 90% (polarographisch ermittelt), was einer Ausbeute von 86% der Theorie entspricht.

Nach dem im Beispiel zur Herstellung von Acethydrazidin beschriebenen Verfahrensweise können auch die in der folgenden Tabelle aufgeführten Verbindungen der Formel

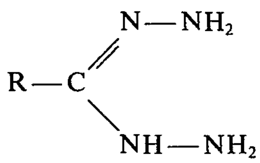

× HCl (Ia)

hergestellt werden:

Tabelle

| Beispiel Nr. | R | Ausbeute (% der Theorie) | Schmelzpunkt (°C) |
|---|---|---|---|
| 2 | $C_2H_5$ | 80 | 109–112 |
| 3 | n-$C_3H_7$ | 76 | 105–115 |
| 4 | iso-$C_3H_7$ | 71 | 126–128 |
| 5 | $ClCH_2$ | 78 | 135–138 |
| 6 | $Cl_3C$ | 69 | 112–115 |

**Patentansprüche**

1. Verfahren zur Herstellung von Hydrazidinen der Formel

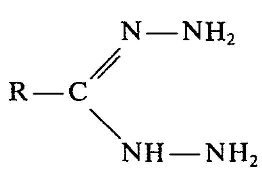

(I)

in welcher

R für gegebenenfalls substituiertes Alkyl, gegebenenfalls substituiertes Cycloalkyl, gegebenenfalls substituiertes Aryl oder gegebenenfalls substituiertes Aralkyl steht,

oder von deren Säureadditionssalzen, ausgehend von den entsprechenden Iminoalkylethern, dadurch

gekennzeichnet, daß man Iminoalkylether der Formel

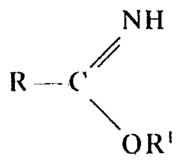

(II)

in welcher

R die oben angegebene Bedeutung hat und
$R^1$ für gegebenenfalls substituiertes Alkyl und gegebenenfalls substituiertes Cycloalkyl steht,

oder deren Säureadditionssalze mit Hydrazinhydrat ($NH_2-NH_2 \times H_2O$) in technischen (wasserhaltigen) Lösungsmitteln bei vermindertem Druck und bei Temperaturen zwischen −80 und +100°C umsetzt.

2. Verfahren nach Anspruch 1, dadurch gekennzeichnet, daß man im Druckbereich von 0,1—800 mbar arbeitet.

3. Verfahren nach Anspruch 1, dadurch gekennzeichnet, daß man bei Drucken zwischen 50 und 300 mbar arbeitet.

4. Verfahren nach Anspruch 1, dadurch gekennzeichnet, daß man bei Temperaturen zwischen −30 und +50°C arbeitet.

5. Verfahren nach Anspruch 1, dadurch gekennzeichnet, daß man auf 1 Mol eines Iminoalkylethers der Formel (II) oder eines entsprechenden Säureadditionssalzes 2—2,5 Mol, vorzugsweise 2—2,3 Mol Hydrazinhydrat einsetzt.

6. Verfahren nach Anspruch 1, dadurch gekennzeichnet, daß man als Ausgangsstoffe Acetiminoethyletherhydrochlorid und Hydrazinhydrat einsetzt.

**Claims**

1. Process for the production of hydrazidines of the formula

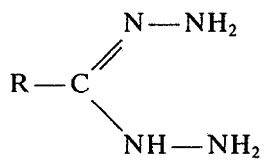

(I)

in which

R represents optionally substituted alkyl, optionally substituted cycloalkyl, optionally substituted aryl or optionally substituted aralkyl,

or acid addition salts thereof, starting from the corresponding iminoalkyl ethers, characterised in that iminoalkyl ethers of the formula

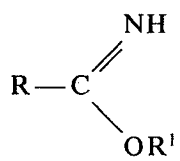

(II)

in which

R has the meaning given above and
$R^1$ represents optionally substituted alkyl and optionally substituted cycloalkyl,

or acid addition salts thereof, are reacted with hydrazine hydrate ($NH_2-NH_2 \times H_2O$) in industrial (water-containing) solvents under reduced pressure and at temperatures between −80 and +100°C.

2. Process according to Claim 1, characterised in that it is carried out under pressures within the range of 0.1—800 mbar.

3. Process according to Claim 1, characterised in that it is carried out under pressures between 50 and 300 mbar.

4. Process according to Claim 1, characterised in that it is carried out at temperatures between −30 and +50°C.

5. Process according to Claim 1, characterised in that 2—2.5 mol, preferably 2—2.3 mol, of hydrazine hydrate are used per mol of an iminoalkyl ether of the formula (II) or of a corresponding acid addition salt.

6. Process according to Claim 1, characterised in that acetiminoethyl ether hydrochloride and hydrazine hydrate are used as the starting materials.

**Revendications**

1. Procédé pour la fabrication d'hydrazidines de formule:

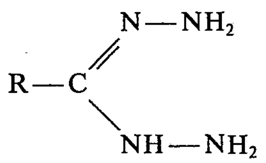

(I)

dans laquelle

R représente un groupe alkyle éventuellement substitué, cycloalkyle éventuellement substitué, aryle éventuellement substitué ou arylalkyle éventuellement substitué,

ou de leurs sels d'addition d'acides, en partant des iminoéthers d'alkyles correspondants, caractérisé en ce que l'on fait réagir des iminoéthers d'alkyles de formule:

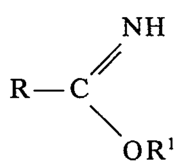

(II)

dans laquelle

R a la signification indiquée ci-dessus et
$R^1$ représente un groupe alkyle éventuellement substitué et cycloalkyle éventuellement substitué,

ou leurs sels d'addition d'acides, avec l'hydrate d'hydrazine ($NH_2-NH_2$, $H_2O$) dans des solvants techniques (aqueux) sous pression réduite et à des températures entre —80 et +100°C.

2. Procédé selon la revendication 1, caractérisé en ce que l'on opère dans le domaine de pression de 0,1—800 mbar.

3. Procédé selon la revendication 1, caractérisé en ce que l'on opère sous des pressions entre 50 et 300 mbar.

4. Procédé selon la revendication 1, caractérisé en ce que l'on opère à des températures entre —30 et +50°C.

5. Procédé selon la revendication 1, caractérisé en ce que l'on utilise 2—2,5 moles d'hydrate d'hydrazine, de préférence 2—2,3 moles, pour une mole d'iminoéther de formule (II) ou d'un sel d'addition d'acide correspondant.

6. Procédé selon la revendication 1, caractérisé en ce que l'on utilise comme matières premières le chlorhydrate d'acétiminoéther d'éthyle et l'hydrate d'hydrazine.